# EUROPEAN PATENT SPECIFICATION

(11) **EP 4 444 179 B1**
(45) Date of publication and mention of the grant of the patent: **30.09.2026**
(21) Application number: 22838951.6
(22) Date of filing: 11.12.2022
(51) Int. Cl.: A61B 5/367, A61B 5/363, A61N 1/362, A61N 1/39

(54) **NON-INVASIVE SYSTEM FOR PREDICTION OF PHYSIOLOGICAL SUBSTRATE ABLATION TARGETS**
NICHTINVASIVES SYSTEM ZUR VORHERSAGE PHYSIOLOGISCHER SUBSTRATABLATIONSZIELE
SYSTÈME NON INVASIF POUR LA PRÉDICTION DE CIBLES D'ABLATION DE SUBSTRAT PHYSIOLOGIQUE

(30) Priority: 12.12.2021 US 202163288633 P; 09.12.2022 US 202218078658
(43) Date of publication of application: 16.10.2024
(73) Proprietor: Biosense Webster (Israel) Ltd., 2066717 Yokneam (IL); University Health Network, Toronto, Ontario M5G 2C4 (CA)
(72) Inventor: BAR-ON, Tal Haim, 2066717 Yokneam (IL); BAR-TAL, Meir, 2066717 Yokneam (IL); HAYAM, Gal, 2066717 Yokneam (IL); SHAPIRA, Einat, 2066717 Yokneam (IL); BEN-DOR, Amir, 2066717 Yokneam (IL); NANTHAKUMAR, Kumaraswamy, Mississauga, Ontario L5H 4M5 (CA); MASSÉ, Stéphane, Quebec Avenue Toronto, Ontario M6P 2T6 (CA); NIRI, Ahmed, Toronto, Ontario M3H 3Z2 (CA)
(74) Representative: Carpmaels & Ransford LLP
(86) International application number: PCT/IB2022/062028
(87) International publication number: WO 2023/105495

(56) References cited:
- WO-A1-2014/183206
- WO-A2-2005/006946
- NIRI AHMED ET AL: "B-PO05-172 AUTOMATED IDENTIFICATION OF VT ABLATION TARGETS: MULTICENTER VALIDATION AND WORKFLOW CHARACTERIZATION", HEART RHYTHM, ELSEVIER, US, vol. 18, no. 8, 27 July 2021 (2021-07-27), XP086706181, ISSN: 1547-5271, DOI: 10.1016/J.HRTHM.2021.06.1091
- JACKSON NICHOLAS ET AL: "Decrement Evoked Potential Mapping : Basis of a Mechanistic Strategy for Ventricular Tachycardia Ablation", CIRCULATION: ARRHYTHMIA AND ELECTROPHYSIOLOGY, vol. 8, no. 6, 1 December 2015 (2015-12-01), United States, pages 1433 - 1442, XP055958657, ISSN: 1941-3149, DOI: 10.1161/CIRCEP.115.003083

## Description

### CROSS-REFERENCE TO RELATED APPLICATIONS

This application claims the benefit of U.S. Provisional Application No. 63/288,633, filed December 12, 2021.

### FIELD OF INVENTION

This invention relates generally to electrophysiological (EP) signals, and specifically to a method for non-invasively detecting abnormal electrical propagation in the heart that can be utilized for screening patients for invasive EP procedures.

### BACKGROUND

Decrement evoked potential (DeEP) is used to identify ventricle tachycardia (VT) substrate ablation targets without the need to induce VT. These potentials are identified by invasive intracardiac mapping protocol. Predicting which patients would be ideal for such physiological mapping protocol, prior to invasive procedure would be practical benefit. A non-invasive tool to predict higher probability of DeEP on 12 lead ECG prior to or during the procedure does not exist and could be of value for the ablating electrophysiologist.

In WO2014183206A1, there is described a system and a method for identifying the arrhythmogenic circuit of a patient or subject. In one embodiment, the method comprises obtaining data for electrograms recorded at various locations of the heart while programmed ventricular pacing with extra stimuli was performed, obtaining decrement values for at least two different locations of the heart using the recorded electrograms, generating at least a portion of a decrement map using the decrement values, and identifying the arrhythmogenic circuit based on electrograms having significant decremental properties.

In WO2005006946A2, there is described a method of delivering a pacing pattern to a heart, measuring a response of the heart to the pacing pattern, the measured response including a series of electrograms representing a plurality of heartbeats of the pacing pattern and calculating diagnostic data from the measured response, wherein the diagnostic data includes data in one of a time domain and a frequency domain.

In *"*B-PO05-172 AUTOMATED IDENTIFICATION OF VT ABLATION TARGETS: MULTICENTER VALIDATION AND WORKFLOW CHARACTERIZATION", Niri Ahmed et al, 27/07/2021, XP086706181, there is described an algorithm that indicates and maps DeEPs by first identifying capture using surface ECG. The algorithm checks for stability of S1 EPs by comparing the last 3 of the 8 morphologies and attributing standard deviation values.

In *"*Decrement Evoked Potential Mapping: Basis of a Mechanistic Strategy for Ventricular Tachycardia Ablation", Jackson Nicholas et al, 01/12/2015, XP05595867, there is described an analysis of whether DeEPs are more likely to colocalize with the diastolic pathways of ventricular tachycardia (VT) circuits.

### SUMMARY

The invention is defined by the appended claims and relates to a system for evaluation of electrical propagation in the heart. Associated methods do not form part of the claimed invention.

A method for evaluation of electrical propagation in the heart is disclosed. The method includes receiving a pacing signal applied to a heart of a patient, the pacing signal comprising (i) a sequence of regular pacing stimuli at slightly lower than sinus rate intervals (S1 paces), and (ii) one or more extra stimuli, premature pacing stimuli at intervals that are shorter than the regular pacing intervals (S2, S3, etc., paces), assessing the envelope of a body-surface ECG component after the regular pacing stimuli, assessing the envelope of a body surface ECG component after the extra stimuli, comparing the assessed component after the extra stimuli to the assessed component after the regular pacing stimuli, and outputting the comparison as an indication to the presence of decrement-evoked potentials (DeEPs) in the patient's heart.

A system for non-invasively detecting abnormal electrical propagation in the heart is disclosed and claimed. The system includes an interface configured to receive a pacing signal applied to a heart of a patient, for example with an existing implantable device such as Automatic Intracardiac Cardioverter-Defibrillator (AICD) or a pacemaker, the pacing signal comprising (i) a sequence of regular pacing stimuli at slightly lower than sinus-rate intervals (S1 paces), and (ii) one or more extra stimuli, premature pacing stimuli at intervals that are shorter than the regular pacing intervals (S2, S3, etc., paces), and a cardiac signal that is sensed, in response to the pacing signal, by a body-surface ECG. The system includes a processor configured to assess the envelope of a body-surface ECG component after the regular pacing stimuli, assess the envelope of a body surface ECG component after the extra stimuli, compare the assessed component after the extra stimuli to the assessed component after the regular pacing stimuli. The interface is further configured to output the comparison as an indication to the presence of DeEPs in the heart of the patient.

The system and method include at least one system component including the measurement of body-surface ECG component characteristics, for example the duration of the QRS envelope, the voltage, the area under the curve, the slopes, the frequency analysis, and the height of the envelope. In the context of the claimed system, the body-surface ECG component characteristic which is measured is the duration of the QRS envelope. The system may be configured to interpret a QRS envelope duration between 0-10msec as indicative of no ablation targets, a QRS envelope duration between 10-50msec as indicative of DeEP positive, and a QRS envelope duration >50msec as indicative of the presence of a prominent DeEP.

### BRIEF DESCRIPTION OF THE DRAWINGS

The present disclosure will be more fully understood from the following detailed description of the embodiments thereof, taken together with the drawings, in which:
FIG. 1 is a schematic illustration of a multi-channel electrocardiograph (ECG) signal measurement system, according to an embodiment;
FIG. 2 illustrates an example of a plurality of body-surface ECG signals indicative of a patient with intra-cardiac decrement-evoked potentials (DeEPs) according to an exemplary embodiment;
FIG.3, collectively depicted as FIG. 3A, FIG. 3B, and FIG. 3C, provide an example of plurality of body-surface ECG signals with QRS widening associated with cases showing presence of DeEP according to an exemplary embodiment;
FIG. 4 illustrates a method of automatic assessment of the body-surface ECG for presence of DeEPs, according to an embodiment;
FIG. 5 illustrates an example of a plurality of body-surface ECG signals for a patient when pacing is performed from the coronary sinus;
FIG. 6 illustrates an example of a plurality of body-surface ECG signals for the patient of FIG. 5 when pacing is performed from the right ventricular apex; and
FIG. 7 illustrates a method of automatic assessment of the body-surface ECG for presence of DeEPs using pacing from different locations.

### DETAILED DESCRIPTION

Cardiac tachycardia, such as ventricular tachycardia (VT) or atrial tachycardia, is a heart rhythm disorder (arrhythmia) caused by abnormal electrical signals in a heart chamber. For example, VT may be caused by abnormal electrical signals in the lower chambers of the heart (ventricles). VT may be caused by local electrophysiological (EP) conduction defects in ventricle tissue, such as in scar tissue. To find and treat such arrhythmogenic locations, for example by ablation, the ventricle may be paced and EP mapped using body-surface ECG and/or intracardiac catheters to identify aberrant tissue locations (e.g., locations demonstrating DeEPs) that may be causing the VT.

In particular, EP mapping may be done in support of a treatment approach to target arrhythmogenic channels and regions, which has been found useful to ablate scar tissue over an entire area of the scar. The motivation behind the ablative treatment is to target poorly coupled ventricle tissue fibers surviving within a developed scar. These bundles are deemed to generate EP paths exhibiting slow conduction (scar isthmuses) believed to be responsible for VT.

In order to perform EP mapping of the pathways and circuits that lead to the tachycardia, in one scenario the tachycardia could be initiated during the EP study. However, VT are often non-inducible or hemodynamically unstable therefore substrate mapping is often necessary. In substrate mapping, characteristics of the tissue in sinus rhythm or ventricular pacing are being related to the arrhythmogenicity of the tissue.

DeEP mapping is a substrate mapping technique. The basis for this method is that ventricular tissue that displays decremental conduction with a decremental extra-stimulus, appears to be more specific to the aberrant VT circuits. The DeEP information is typically presented as a DeEP data layer overlaid on an EP map of the ventricle.

In the disclosed technique, the ventricle is paced with short sequences of regular pulses slightly faster than sinus rhythm. The short sequences end with one or more pulses (typically three at most) having shorter inter-pulse intervals. Common notation defines this as pacing with a train of S1 paces (usually, but not always, having cycle length of 600msec) followed by one or more decremental extra-stimuli (S2, S3, etc.) delivered shortly after (e.g., 20 milliseconds after) a period of time (commonly referred to as the refractory period) that a new action potential cannot normally be initiated, so as to stimulate the looked for arrhythmogenic response in aberrant tissue.

In the context of this description the wording "regular pulses faster than sinus rhythm" covers pulses that are equidistant up to a predefined variation. For example, a sequence of pulses with an interval 600±6 milliseconds are included in the definition. More generally, a sequence of pulses with any given equidistant interval (e.g., smaller or larger than 600 milliseconds, such as 500 msec or 700 msec) up to the predefined variation are considered hereinafter "regular pulses faster than sinus rhythm". Typically, the predefined variation is limited to an order of 1% of the specified equidistant interval. The respective decremental extra-stimuli are defined with respect to the above equidistant interval. For example, if the regular pacing pulses are done with a period of 600 milliseconds, the one or more short-interval pacing stimuli can be applied at 400 milliseconds intervals following the regular rate stimulus. The terms "pulses" and "stimuli" are used interchangeably herein.

The above described sequence simulates an early beat (premature ventricle contraction, PVC), for invoking DeEPs in an arrhythmogenic tissue. In general, early beat is something relatively common. Only if the early beat encounters an arrhythmogenic tissue, like a tissue that shows slower conduction as a result of the early beat, an aberrant VT circuit can be initiated and maintained.

In response to the regular sinus rate pacing pulses, evoked potentials occur one each with a normal time delay after the stimulus. This normal delay, between a pacing pulse and the resulting evoked potential, is called hereinafter "first time delay." This portion of the EP mapping method is sufficient for characterizing normal tissue.

In response to the extra-stimuli, the evoked potential may be delayed as the wave speed is decremented (relatively to the first-time delay). The areas where there is a meaningful relative delay (e.g., more than 10 msec) are considered to be more specific to an aberrant VT circuit. The larger delay than the first-time delay, if occurring, of these evoked potentials, relative to the corresponding extra-stimuli pacing pulses, is called hereinafter "second time delay." This delay, present on the surface of the heart, can be measured on the body-surface ECG, for example, in the form of QRS widening.

A processor analyzes resulting body-surface ECG signals to predict the presence of DeEPs. In some embodiments, the processor performs the following steps: generating regular paces (S1) and then one or more extra stimuli that potentially cause DeEPs; extracting body-surface ECG responses to S1 and correlate these responses to each other to confirm rhythm stability; extracting the ECG response to the last S1 pace, called hereinafter the S1 QRS component, and extracts the ECG response to the first extrastimuli (S2 pace), called hereinafter the S2 QRS component; calculating the widths of the S1 QRS component and S2 QRS component; and comparing the width of the S2 QRS component to the width of the S1 QRS component to give indication to the presence of DeEPs
More generally, the processor can estimate a degree of correlation by any other methods known in the art, such as by using machine learning, and define a scoring, to find a correlation with a highest scoring. For example, the scoring may be based on a prespecified metric, such as L1.

Typically, the processor is programmed in software containing a particular algorithm that enables the processor to conduct each of the processor-related steps and functions outlined above.

By applying automated DeEP predictive algorithm to assess presence of local cardiac (e.g., ventricle) tissue locations inducing VT, the disclosed non-invasive cardiac diagnostic method may improve the safety and value of diagnostic catheterization procedures.

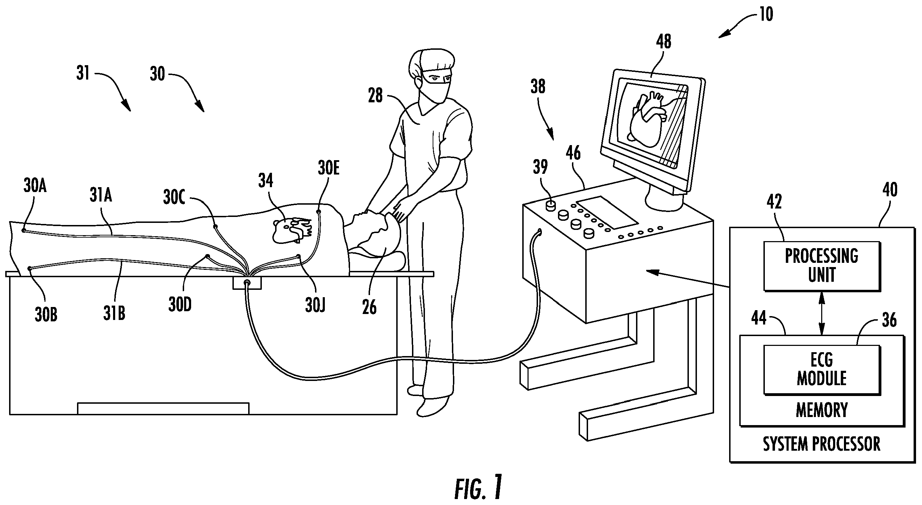

FIG. 1 illustrates a schematic illustration of a multi-channel electrocardiograph (ECG) signal measurement system 10, according to an embodiment of the present invention.

For simplicity and clarity, the following description, except where otherwise stated, assumes an investigative procedure wherein system 10 senses body surface electrical signals from a heart 34 of a subject 26.

In order to sense body-surface electrical signals, electrodes 30A, 30B, 30C, . . . are attached to the skin of subject 26 by respective leads 31A, 31B, 31C, . . . . In the present disclosure electrodes 30A, 30B, 30C, . . . are collectively termed electrodes 30, and leads 31A, 31B, 31C, ... are collectively termed leads 31. In a typical ECG procedure where, only body-surface electrical signals are measured, there are ten electrodes 30 attached to the skin of subject 26 in standard positions: right arm, left arm, right leg, left leg., as well as six electrodes in the region of heart 34. In FIG. 1 four electrodes 30A, 30B, 30C, and 30D, are illustrated, and are assumed to be respectively attached to the right leg, left leg, right arm, and the left arm of subject 26. For clarity, only two electrodes 30E and 30J of the six electrodes attached in the region of heart 34, for the typical ECG procedure referred to above, are shown in FIG. 1. However, there may be more than ten, or fewer than ten, electrodes 30 in some ECG procedures, and there is no restriction on the number of electrodes 30 for embodiments of the present invention. It will be understood that each electrode 30 defines a respective channel of system 10.

System 10 may be controlled by a system processor 40, comprising a processing unit 42 communicating with a memory 44. Processor 40 is typically mounted in a console 46, which comprises operating controls 38, typically including a pointing device 39 such as a mouse or trackball, that professional 28 uses to interact with the processor. The processor uses software, including an ECG module 36, stored in memory 44, to operate system 10. Results of the operations performed by processor 40 are presented to the professional on a display 48, which typically presents a graphic user interface to the user, a visual representation of the ECG signals sensed by electrodes 30, and/or an image or map of heart 34 while it is being investigated. The software may be downloaded to processor 40 in electronic form, over a network, for example, or it may, alternatively or additionally, be provided and/or stored on non-transitory tangible media, such as magnetic, optical, or electronic memory.

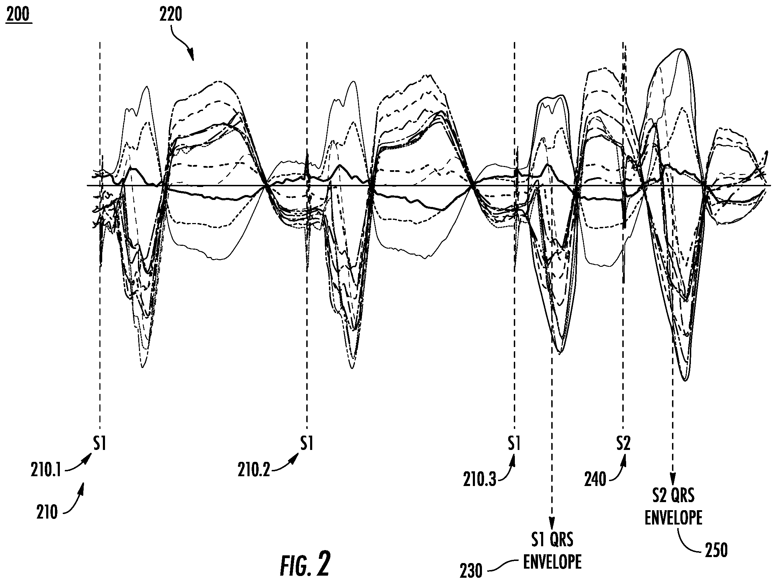

ECG module 36 is coupled to receive electrical signals from electrodes 30. The module is configured to analyze the signals and may present the results of the analysis in a standard ECG format, typically a graphical representation moving with time, on display 48. The structure and operation of module 36 is described in more detail below with respect to FIGS. 2-4.

The example illustration shown in Fig. 1 is chosen purely for the sake of conceptual clarity. Other types of electrophysiological sensing catheter geometries may be employed.

By utilizing the body-surface ECG, a non-invasive measuring technique, the present system and method provide a way for a physician to know before an invasive procedure occurs whether a DeEP in a ventricle exists, allowing the physician to predict the need for an invasive DeEP procedure. The physician can screen patients, many of whom are vulnerable, thereby allowing for better screening of the patients before intracardiac techniques are utilized.

FIG. 2 illustrates an example of a plurality of body-surface ECG signals 200 indicative of a patient with intra-cardiac DeEPs according to an exemplary embodiment. The body-surface ECG signals include the individual electrode signals and are discussed herein collectively as would be understood by those possessing an ordinary skill in the art. As illustrated, an S1 pace 210 is periodically (illustrated in FIG. 2 as S1 pace signals 210.1, 210.2, 210.3, collectively referred to as S1 pace 210) provided and the body-surface ECG signals 220 are measured. While the description identifies the S1 pace 210, a normal sinus rhythm may be used. From the body-surface ECG signals 220, the S1 body-surface ECG component 230, such as QRS envelope, is measured.

A S2 extrastimulus 240 is provided and the body-surface ECG signals 220 are measured. While the description identifies the S2 extrastimulus 240, a premature ventricular contraction (PVC) may be used. From the body-surface ECG signals 220, the S2 body-surface ECG component 250, such as the QRS envelope, is measured.

The envelope of the body-surface ECG component 230 (for S1), 250 (for S2), such as the QRS, for example, is assessed for both the S1 pace 210 and the S2 extrastimulus 240. Parameters of the envelope of the body-surface ECG component, such as the QRS envelopes 230 (for S1), 250 (for S2), may be compared. These parameters may also include the duration, voltage, area under the curve, slopes, frequency analysis, and the height of the envelope, for example. Using the comparison of the parameters, by examining at least one of the parameter comparisons, the system and method predict existence of physiological substrate ablation targets for ventricular tachycardia (VT). Typically, to be clinically significant, the difference in the comparison of the parameters should be well above several units.

By way of example, body-surface ECG prediction can be calculated by subtracting the width of the ECG envelope of S1, such as S1 QRS envelop 230, from width of the ECG envelope of S2, such as S2 QRS envelop 250. By way of example, an ECG QRS widening of envelope width above a certain threshold (e.g., more than 20 milliseconds) indicates the presence of DeEP targets. This system and method predict physiological substrate ablation targets for ventricular tachycardia (VT).

As would be understood, checks for the stability of the plurality of S1 responses may also be calculated. The body-surface ECG signals 220 are measured after each of the S1 paces 210 (such as after individual S1 paces illustrated as 210.1, 210.2, 210.3). From the body-surface ECG signals 220, the S1 body-surface ECG component, such as QRS envelope 230, is measured. The parameters of the envelopes resulting from S1 paces 210 (such as after individual S1 paces illustrated as 210.1, 210.2, 210.3) may be compared. This comparison may provide the stability of the S1 response. If the standard deviation of the comparison is below a given threshold, the timing of response is considered well defined and useful. The threshold may be set by a clinician, for example. The threshold is a guide to determine if the beats are similar, indicating stability of the measurement, such as singularity to beats. In one example, 0.85 threshold may be used to identify a stable configuration. As would be understood, 0.85 is exemplary, and other stability measurements such as 08., 0.75, 0.9, or above could indicate a stable configuration for the measurement. The threshold may also be altered by the clinician to meet the stability desired during a given measurement. An average of the S1 response, such as an average of three S1 responses, or the latest S1 response, may be used in a subsequent calculation of QRS widening or other parameters.

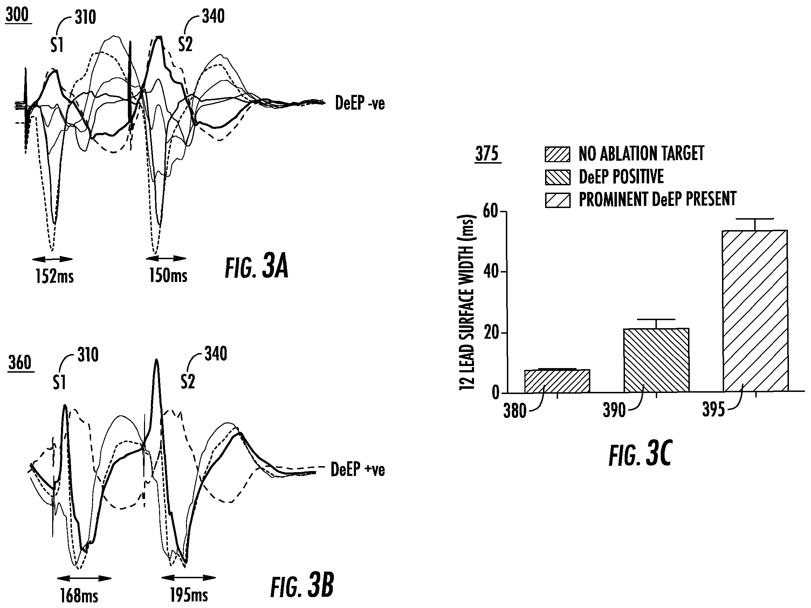

FIG.3, collectively depicted as FIG. 3A, FIG. 3B, and FIG. 3C, provide an example of plurality of body-surface ECG signals with QRS widening associated with cases showing the presence of DeEP according to an exemplary embodiment. As in FIG. 2, the signals are measured with a body-surface ECG 220 using S1 pacing 210 and an S2 extrastimulus 240. Again, the body-surface ECG signals include the individual electrode signals and are discussed herein collectively as would be understood by those possessing an ordinary skill in the art. Figure 3C illustrates the relation between surface QRS widening (the y-axis) versus 3 DeEP classifications.

Referring to FIG. 3A, there is illustrated the body-surface signals 300 for both the S1 310 and S2 340 pacing configuration. Using the surface width of the QRS envelope as the parameter of interest, the surface width after the S1 pacing signal 310 is illustrated as 152 msec and the surface width after the S2 extrastimulus 340 is illustrated as 150 msec. In this scenario depicted in FIG. 3A, the difference is effectively 0 msec indicating that no ablation target is present.

Referring to FIG. 3B, there is illustrated the body-surface signals 360 for both the S1 310 and S2 340 pacing configuration. Using the surface width of the QRS envelope as the parameter of interest, as in FIG. 3A, the surface width after the S1 pacing signal 310 is illustrated as 168 msec and the surface width after the S2 extrastimulus 340 is illustrated as 195 msec. In this scenario depicted in FIG. 3B, the difference is effectively 30 msec indicating that a prominent DeEP present.

Referring to FIG. 3C, there is illustrated a plot 375 of the relation between surface QRS widening (the y-axis) versus 3 DeEP classifications categories. A first category of "No ablation targets" 380 is illustrated having DeEP between 0-10msec. A second category of "DeEP positive" 390 is illustrated having DeEP between 10 and 50msec. A third category of "Prominent DeEP" 395 is illustrated having DeEP greater than 50msec.

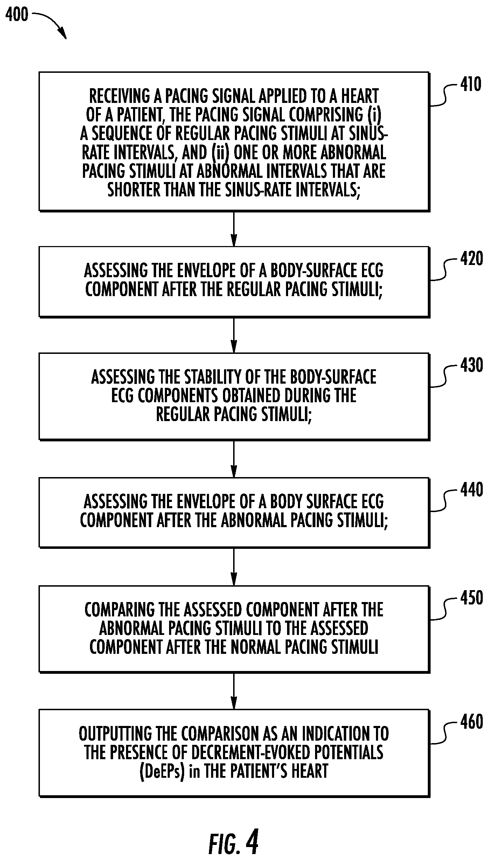

FIG. 4 illustrates the basic steps of a method 400 for non-invasively detecting abnormal electrical propagation in the heart according to an embodiment.

Method 400 includes at step 410 receiving a pacing signal applied to a heart of a patient. The pacing signal includes (i) a sequence of regular pacing stimuli slightly faster than sinus rhythm, and (ii) one or more abnormal pacing stimuli at abnormal intervals that are shorter than the regular-rate intervals.

At step 420, the method 400 includes assessing the envelope of a body-surface ECG component after the regular pacing stimuli. The assessed component is the duration of the QRS envelope, a body surface ECG component duration, the voltage, the area under the curve, the slopes, the frequency analysis and/or the height of the envelope, for example.

At step 430, the method 400 includes assessing the stability of the body-surface ECG components obtained during the regular pacing stimuli.

At step 440, the method 400 includes assessing the envelope of a body surface ECG component after the abnormal pacing stimuli. In an example, the assessed component being identical to the assessed component in step 420. The assessed component is the duration of the QRS envelope, a body surface ECG component duration, the voltage, the area under the curve, the slopes, the frequency analysis and/or the height of the envelope, for example.

At step 450, the method 400 includes comparing the assessed component after the abnormal pacing stimuli to the assessed component after the regular pacing stimuli. For example, the difference in the width of the QRS signal of the two signals.

At step 460, the method 400 includes outputting the comparison as an indication to the presence of DeEPs in the patient's heart.

The system and method provide detection of patients who have substrate ablation targets based on 12 lead ECG, prior to the ablation procedure. Additionally, this prediction rule, based on its association with arrhythmogenic potentials, may be able to predict which ambulatory patients who have not manifest VT, may be prone to develop VT. This concept should be validated in larger data sets and is ideal for training and testing using surface ECG envelopes with artificial intelligence algorithms.

The example flow chart shown in FIG. 4 is chosen purely for the sake of conceptual clarity. The present embodiment may also comprise additional steps of the algorithm. This and other possible steps are omitted from the disclosure herein purposely in order to provide a more simplified flow chart.

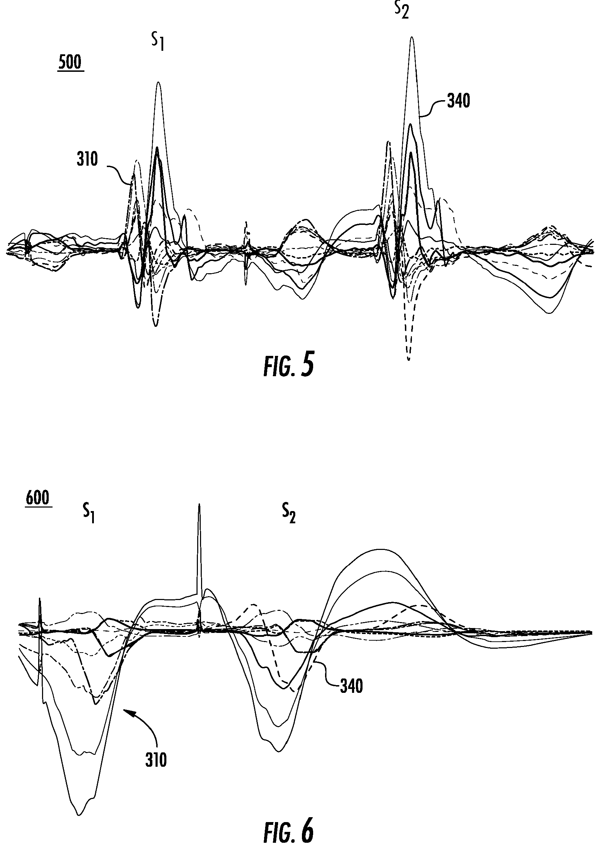

The pacing may occur from different locations and/or the location of the pacing signal may be varied. FIGs. 5 and 6 are illustrative of the results of a configuration of the 12-leads BS ECG when pacing is performed from different locations. By way of example, for a given patient, multiple locations of the pacing signal may be used in recording the signals (measurements). For example, in FIG. 5 the pacing is provided from the coronary sinus with the resulting recorded body-surface ECG signals, and in FIG. 6 the pacing is provided from the right ventricular apex with the resulting recorded body-surface ECG signals. In each of FIGs. 5 and 6 the characteristics of the QRS envelope, or the extent that the QRS envelop changes can be different even in the case of the same patient.

FIG. 5 illustrates an example of a plurality of body-surface ECG signals 500 for a patient when pacing is performed from the coronary sinus. Within FIG. 5 there is illustrated the characteristics of the QRS envelope repeated over two beats. Again, the body-surface ECG signals include the individual electrode signals and are discussed herein collectively as would be understood by those possessing an ordinary skill in the art. This QRS envelop includes S1 and S2 as described above. The envelope of the body-surface ECG component for S1 and for S2, such as the QRS, for example, is assessed for both the S1 pace and the S2 extrastimulus as described. Parameters of the envelope of the body-surface ECG component, such as the QRS envelopes S1, S2, may be compared. The body-surface signals 500 for both the S1 310 and S2 340 pacing configuration are illustrated. As described above, using the surface width of the QRS envelope as the parameter of interest, the surface width after the S1 pacing signal 310 may be measured and the surface width after the S2 extrastimulus 340 may be measured. In this scenario, the difference may be compared as described to determine if an ablation target is present.

FIG. 6 illustrates an example of a plurality of body-surface ECG signals 600 for the patient of FIG. 5 when pacing is performed from the right ventricular apex. Again, the body-surface ECG signals include the individual electrode signals and are discussed herein collectively as would be understood by those possessing an ordinary skill in the art. Within FIG. 6 there is illustrated the characteristics of the QRS envelope repeated over two beats. This QRS envelop includes S1 and S2 as described above. The envelope of the body-surface ECG component for S1 and for S2, such as the QRS, for example, is assessed for both the S1 pace and the S2 extrastimulus as described. Parameters of the envelope of the body-surface ECG component, such as the QRS envelopes S1, S2, may be compared. The body-surface signals 600 for both the S1 and S2 pacing configuration. As described above, using the surface width of the QRS envelope as the parameter of interest, the surface width after the S1 pacing signal may be measured and the surface width after the S2 extrastimulus may be measured. In this scenario, the difference may be compared as described to determine if an ablation target is present.

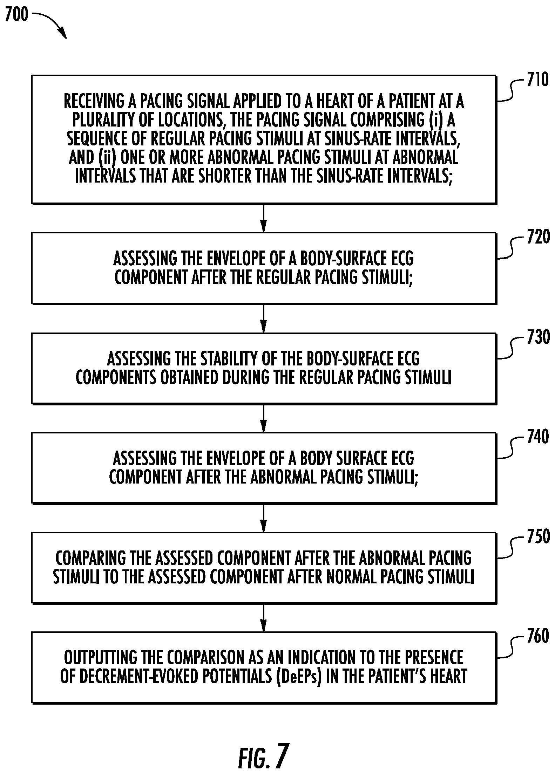

FIG. 7 illustrates a method 700 of automatic assessment of the body-surface ECG for presence of DeEPs using pacing from different locations. Similar to the method described above with respect to FIG. 4, Method 700 includes at step 710 receiving a pacing signal applied to a heart of a patient at different locations. The pacing signal includes (i) a sequence of regular pacing stimuli slightly faster than sinus rhythm, and (ii) one or more abnormal pacing stimuli at abnormal intervals that are shorter than the regular-rate intervals. The pacing signal may be provided at different locations about the heart of a patient.

At step 720, the method 700 includes assessing the envelope of a body-surface ECG component after the regular pacing stimuli. The assessed component is the duration of the QRS envelope, a body surface ECG component duration, the voltage, the area under the curve, the slopes, the frequency analysis and/or the height of the envelope, for example.

At step 730, the method 700 includes assessing the stability of the body-surface ECG components obtained during the regular pacing stimuli.

At step 740, the method 700 includes assessing the envelope of a body surface ECG component after the abnormal pacing stimuli. In an example, the assessed component being identical to the assessed component in step 720. The assessed component is the duration of the QRS envelope, a body surface ECG component duration, the voltage, the area under the curve, the slopes, the frequency analysis and/or the height of the envelope, for example.

At step 750, the method 700 includes comparing the assessed component after the abnormal pacing stimuli to the assessed component after the regular pacing stimuli. For example, the difference in the width of the QRS signal of the two signals.

Method 700 may be repeated for additional pacing locations to further measure the difference in the width of the QRS signal of the two signals under the presence of differing pacing signals.

At step 760, the method 700 includes outputting the comparison as an indication to the presence of DeEPs in the patient's heart based on the measured QRS signals for the different pacing locations.

The system and method provide detection of patients who have substrate ablation targets based on a 12 lead ECG, prior to the ablation procedure. Additionally, this prediction rule, based on its association with arrhythmogenic potentials, may be able to predict which ambulatory patients who have not manifested VT, may be prone to develop VT. This concept should be validated in larger data sets and is ideal for training and testing using surface ECG envelopes with artificial intelligence algorithms.

The example flow chart shown in FIG. 7 is chosen purely for the sake of conceptual clarity. The present embodiment may also comprise additional steps of the algorithm. This and other possible steps are omitted from the disclosure herein purposely in order to provide a more simplified flow chart.

Although the embodiments described herein mainly address cardiac diagnostic applications, the methods and systems described herein can also be used in other medical applications.

It will be appreciated that the embodiments described above are cited by way of example, and that the present invention is not limited to what has been particularly shown and described hereinabove. Rather, the scope of the present invention is defined by the appended claims.

## Claims

1. A system (10) for evaluation of electrical propagation in the heart, the system comprising:
an interface configured to receive:
a pacing signal applied to a heart of a patient; and
a cardiac signal that is sensed, in response to the pacing signal, by a body-surface ECG; and
a processor (40), which is configured to:
receive the pacing signal and the cardiac signal from the interface, the pacing signal comprising (i) a sequence of regular pacing stimuli (210) at regular intervals, followed by (ii) one or more extra stimuli (240) at abnormal intervals that are shorter than the intervals of the regular pacing stimuli;
**characterised in that** the processor is further configured to:
assess the envelope of a body-surface ECG (220) response to the regular pacing stimuli, wherein assessing the envelope comprises assessing the duration of a QRS envelope of the body-surface ECG response to the regular pacing stimuli;
assess the envelope of a body-surface ECG (220) response to the extra stimuli, wherein assessing the envelope comprises assessing the duration of a QRS envelope of the body-surface ECG response to the extra stimuli;
compare the duration of the QRS envelope of the body-surface ECG response to the extra stimuli to the duration of the QRS envelope of the body-surface ECG response to the regular pacing stimuli; and
output the comparison, using the interface, as an indication to the presence of decrement-evoked potentials (DeEPs) in the patient's heart.

2. The system of claim 1, wherein the processor (40) is further configured to assess the stability of the body surface ECG components obtained during the regular pacing stimuli.

3. The system of claim 1, wherein the duration of the QRS envelope of the body-surface ECG response to the extra stimuli being greater than the duration of the QRS envelope of the body-surface ECG response to the regular pacing stimuli by between 0-10msec is indicative of no ablation targets.

4. The system of claim 1, wherein the duration of the QRS envelope of the body-surface ECG response to the extra stimuli being greater than the duration of the QRS envelope of the body-surface ECG response to the regular pacing stimuli by between 10-50msec is indicative of DeEP positive.

5. The system of claim 1, wherein the duration of the QRS envelope of the body-surface ECG response to the extra stimuli being greater than the duration of the QRS envelope of the body-surface ECG response to the regular pacing stimuli being greater than 50msec is indicative of the presence of a prominent DeEP.

## Patentansprüche

1. System (10) für eine Bewertung einer elektrischen Ausbreitung in dem Herzen, das System umfassend:
eine Schnittstelle, die konfiguriert ist, um zu empfangen:
ein an ein Herz eines Patienten angelegtes Stimulationssignal; und
ein Herzsignal, das als Reaktion auf das Stimulationssignal durch ein Körperoberflächen-EKG erfasst wird; einen Prozessor (40), der konfiguriert ist zum:
Empfangen des Stimulationssignals und des Herzsignals von der Schnittstelle, das Stimulationssignal umfassend (i) eine Sequenz von regelmäßigen Stimulationsimpulsen (210) in regelmäßigen Intervallen, gefolgt durch (ii) einen oder mehrere Extraimpulse (240) in abnormalen Intervallen, die kürzer als die Intervalle der regelmäßigen Stimulationsimpulse sind;
**dadurch gekennzeichnet, dass** der Prozessor ferner konfiguriert ist zum:
Beurteilen der Hüllkurve einer Antwort des Körperoberflächen-EKG (220) auf die regelmäßigen Stimulationsimpulse, wobei das Beurteilen der Hüllkurve das Bewerten der Dauer einer QRS-Hüllkurve der Antwort des Körperoberflächen-EKG auf die regelmäßigen Stimulationsimpulse umfasst;
Beurteilen der Hüllkurve einer Antwort des Körperoberflächen-EKG (220) auf die Extraimpulse, wobei das Bewerten der Hüllkurve das Bewerten der Dauer einer QRS-Hüllkurve der Antwort des Körperoberflächen-EKG auf die Extraimpulse umfasst;
Vergleichen der Dauer der QRS-Hüllkurve der Antwort des Körperoberflächen-EKG auf die Extraimpulse mit der Dauer der QRS-Hüllkurve der Antwort des Körperoberflächen-EKG auf die regelmäßigen Stimulationsimpulse; und
Ausgeben des Vergleichs, unter Verwendung der Schnittstelle, als einen Hinweis auf das Vorliegen von dekrementevozierten Potenzialen (DeEPs) in dem Herzen des Patienten.

2. System nach Anspruch 1, wobei der Prozessor (40) ferner konfiguriert ist, um die Stabilität der Körperoberflächen-EKG-Komponenten zu beurteilen, die während der regelmäßigen Stimulationsimpulse erhalten werden.

3. System nach Anspruch 1, wobei die Dauer der QRS-Hüllkurve der Antwort des Körperoberflächen-EKG auf die Extraimpulse, die um zwischen 0-10 ms größer als die Dauer der QRS-Hüllkurve der Antwort des Körperoberflächen-EKG auf die regelmäßigen Stimulationsimpulse ist, auf keine Ablationsziele hinweist.

4. System nach Anspruch 1, wobei die Dauer der QRS-Hüllkurve der Antwort des Körperoberflächen-EKG auf die Extraimpulse, die um zwischen 10-50 ms größer als die Dauer der QRS-Hüllkurve der Antwort des Körperoberflächen-EKG auf die regelmäßigen Stimulationsimpulse ist, auf DeEP-positiv hinweist.

5. System nach Anspruch 1, wobei die Dauer der QRS-Hüllkurve der Antwort des Körperoberflächen-EKG auf die Extraimpulse, die größer als die Dauer der QRS-Hüllkurve der Antwort des Körperoberflächen-EKG auf die regelmäßigen Stimulationsimpulse ist, die größer als 50 ms ist, auf das Vorliegen eines ausgeprägten DeEP hinweist.

## Revendications

1. Système (10) destiné à l'évaluation de propagation électrique dans le cœur, le système comprenant :
une interface configurée pour recevoir :
un signal de stimulation appliqué à un cœur d'un patient ; et
un signal cardiaque qui est capté, en réponse au signal de stimulation, par un ECG de surface corporelle ; et un processeur (40), qui est configuré pour :
recevoir le signal de stimulation et le signal cardiaque en provenance de l'interface, le signal de stimulation comprenant (i) une séquence de stimuli de stimulation réguliers (210) à des intervalles réguliers, suivis par (ii) un ou plusieurs stimuli supplémentaires (240) à des intervalles anormaux qui sont plus courts que les intervalles des stimuli de stimulation réguliers ;
**caractérisé en ce que** le processeur est configuré en outre pour :
évaluer l'enveloppe d'une réponse de l'ECG de surface corporelle (220) aux stimuli de stimulation réguliers, dans lequel l'évaluation de l'enveloppe comprend l'évaluation de la durée d'une enveloppe QRS de la réponse d'ECG de surface corporelle aux stimuli de stimulation réguliers ;
évaluer l'enveloppe d'une réponse de l'ECG de surface corporelle (220) aux stimuli supplémentaires, dans lequel l'évaluation de l'enveloppe comprend l'évaluation de la durée d'une enveloppe QRS de la réponse d'ECG de surface corporelle aux stimuli supplémentaires ;
comparer la durée de l'enveloppe QRS de la réponse d'ECG de surface corporelle aux stimuli supplémentaires à la durée de l'enveloppe QRS de la réponse d'ECG de surface corporelle aux stimuli de stimulation réguliers ; et
sortir la comparaison, à l'aide de l'interface, en tant qu'indication de la présence de potentiels évoqués décrémentiels (DeEP) dans le cœur du patient.

2. Système selon la revendication 1, dans lequel le processeur (40) est configuré en outre pour évaluer la stabilité des composantes d'ECG de surface corporelle obtenues pendant les stimuli de stimulation réguliers.

3. Système selon la revendication 1, dans lequel la durée de l'enveloppe QRS de la réponse d'ECG de surface corporelle aux stimuli supplémentaires étant supérieure à la durée de l'enveloppe QRS de la réponse d'ECG de surface corporelle aux stimuli de stimulation réguliers d'une durée comprise de 0 à 10 msec indique une absence de cibles d'ablation.

4. Système selon la revendication 1, dans lequel la durée de l'enveloppe QRS de la réponse d'ECG de surface corporelle aux stimuli supplémentaires étant supérieure à la durée de l'enveloppe QRS de la réponse d'ECG de surface corporelle aux stimuli de stimulation réguliers d'une durée comprise de 10 à 50 msec indique un DeEP positif.

5. Système selon la revendication 1, dans lequel la durée de l'enveloppe QRS de la réponse d'ECG de surface corporelle aux stimuli supplémentaires étant supérieure à la durée de l'enveloppe QRS de la réponse d'ECG de surface corporelle aux stimuli de stimulation réguliers d'une durée supérieure à 50 msec indique la présence d'un DeEP marqué.
